# EUROPEAN PATENT APPLICATION

(11) **EP 4 531 056 A1**
(43) Date of publication of application: **02.04.2025**
(21) Application number: 24201849.7
(22) Date of filing: 23.09.2024
(51) Int. Cl.: G16H 50/20, G16H 50/30, G16H 50/70, A61B 5/00, G06N 3/091

(54) **ACTIVE LEARNING ON BIOLOGICAL SOUNDS FOR DETERMING PRESENCE OF MEDICAL CONDITION**

(30) Priority: 26.09.2023 US 202318372934
(71) Applicant: Robert Bosch GmbH, 70442 Stuttgart (DE)
(72) Inventor: Ghaffarzadegan, Shabnam, Livermore, CA 94550 (US); Aracri, Joseph, Pittsburgh, PA 15212 (US); Wu, Ho-Hsiang, Morrisville, NC 27560 (US); Shields, Kelly, Pittsburgh, PA 15222 (US); Bondi, Luca, Pittsburgh, PA 15218 (US); Das, Samarjit, Wexford, PA 15090 (US)

(57) **Abstract**

Methods and systems for training an audio-based machine learning model to predict a health condition based on biological sounds emitted by a person. Audio data corresponding to biological sounds produced by the person is generated from a microphone. The audio data is segmented into a plurality of segments, each segment associated with a respective sound event. An audio-based machine learning model is executed on the plurality of segments. The audio-based machine learning model is configured to output, for each segment, a label of a medical condition and an associated a confidence score. The model is trained via active learning, in which a subset of the plurality of segments are selected based on their confidence score being below a threshold, and provided to a human for annotation.

## Description

### TECHNICAL FIELD

The present disclosure relates to machine learning systems trained with active learning on biological sounds emitted by a human body for determining the presence of an associated medical condition.

### BACKGROUND

The usage of data-driven approaches in detection of medical conditions can include screening and diagnosis, patient classification, monitoring, and treatment. Various data resources and modalities have been utilized including medical history documents, patterns of symptoms, environmental data, and questionnaires. Most studies in this space utilize smaller datasets due to the difficulty in collecting data on the target population and the cost in terms of time and resources. Developing robust traditional machine learning applications or deep-learning based approaches to flag medical conditions becomes difficult with such sparse data, and poses a critical concern of an experiment's validity and generalizability.

### SUMMARY

In an embodiment, a method for training an audio-based machine learning model with active learning is provided. The method includes: receiving audio data corresponding to biological sounds produced by a body of a patient; segmenting the audio data into a plurality of segments; executing an audio-based machine learning model on the plurality of segments, wherein the audio-based machine learning model is configured to output, for each segment, a label of a medical condition and an associated a confidence score; storing the labels and associated confidence scores in storage; and training the audio-based machine learning model via active learning. The training includes: retrieving a subset of the plurality of segments from the storage; receiving annotations from a human annotator, wherein the annotations are associated with medical conditions; and training the audio-based machine learning model based on the annotations until convergence to yield a trained sound-based machine learning model configured to output a predicted medical condition associated with input biological sounds.

In another embodiment a system includes a processor and a non-transitory memory coupled to the processor comprising instructions executable by the processor, the processor operable when executing the instructions to: receive audio data generated from a microphone and corresponding to biological sounds produced by a body of a patient; segment the audio data into a plurality of segments, execute an audio-based machine learning model on the plurality of segments, wherein the audio-based machine learning model is configured to output, for each segment, a label of a medical condition and an associated a confidence score; store the labels and associated confidence scores; and train the audio-based machine learning model via active learning. The training includes retrieving a subset of the plurality of segments; receiving annotations from a human annotator, wherein the annotations are associated with medical conditions; and training the audio-based machine learning model based on the annotations until convergence to yield a trained sound-based machine learning model configured to output a predicted medical condition.

In other embodiments, a computer-readable non-transitory storage medium embodies software that is operable to perform the steps disclosed herein.

### BRIEF DESCRIPTION OF THE DRAWINGS

FIG. 1 is a diagram of an active learning system configured to carry out large-scale audio collection and annotation of audio data via active learning, according to an embodiment.
FIG. 2 illustrates a system-level flowchart of a system for enabling the large-scale audio data collection and annotation of audio data for healthcare applications via an active learning framework, according to an embodiment.
FIG. 3 illustrates a flowchart of a method for training a machine learning model via the active learning system, according to an embodiment.
FIG. 4 shows a schematic of a deep neural network with nodes in an input layer, multiple hidden layers, and an output layer, according to an embodiment.
FIG. 5 illustrates an example computer system for carrying out at least portions of the methods and systems described herein.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative bases for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical application. Various combinations and modifications of the features consistent with the teachings of this disclosure, however, could be desired for particular applications or implementations.

"A", "an", and "the" as used herein refers to both singular and plural referents unless the context clearly dictates otherwise. By way of example, "a processor" programmed to perform various functions refers to one processor programmed to perform each and every function, or more than one processor collectively programmed to perform each of the various functions.

Asthma is a chronic respiratory medical condition affecting more than 300 million children and adults worldwide with a high hospitalization rate during acute episodes. Historically, the definition of asthma has been subjective; however, there is an agreement in the physiological response including a tightening of the muscles around small airways combined with inflammation in the lung leading to airway obstruction. Common asthma symptoms include coughing, wheezing, shortness of breath, and chest tightness. These symptoms may appear intermittently, often worse at night, and are triggered by various conditions such as exercise, allergen and irritant exposure, viral infections (e.g. colds), changes in weather, etc.

Diagnosis of asthma is often based on assessing a patient's medical history, identifying patterns of respiratory symptoms, and more recently blood biomarkers. Additionally, clinicians may utilize a questionnaire, which is simple and convenient, to aid in an asthma diagnosis. World-wide initiatives including the Global Initiative of Asthma (GINA) are using sample questionnaires to collect data on the frequency and severity of lung sound abnormalities (e.g. wheezing and coughing). Despite health records, questionnaires, and patients' descriptions, a true asthma diagnosis remains challenging due to a lack of accepted universal rules. An asthma diagnosis may vary due to clinicians' experience level, geographic area, and due to the disease and manifestations changing over time. A true asthma diagnosis is especially challenging in young children where questionnaires cannot be used as a reliable tool of choice.

Though asthma cannot be cured, a timely diagnosis along with proper medications and awareness can control the disease. However, untreated asthma lessens a patient's quality of life and can lead to major healthcare costs. Self-awareness and monitoring are an asthma patient's best tools in reducing the impact of the disease on their life. Self-monitoring is subjective, particularly with patients that have caregivers (e.g. children), and can lead to a misdiagnosis and ultimately poor treatment. An automatic, data-driven, at-home asthma condition monitoring system that is non-invasive, cost-effective, and patient-friendly can play an important role in disease control and promote remote medical care which has become increasingly popular since the COVID-19 Pandemic.

The usage of data-driven approaches in asthma detection can be categorized into four major groups: screening and diagnosis, patient classification, monitoring, and treatment. Various data resources and modalities have been utilized for each including lung sounds from auscultation, medical history documents, patterns of symptoms, environmental data, and questionnaires. Most studies in this space utilize smaller datasets due to the difficulty in collecting data on the target population and the cost in terms of time and resources. Developing robust traditional machine learning applications or deep-learning based approaches to flag an asthma condition becomes difficult with such sparse data, and poses a critical concern of an experiment's validity and generalizability. Audio data collection can be non-invasive, cost effective, and patient friendly. Hence, a noncontact, automatic lung sound abnormality assessment system can be seamlessly integrated into the patient's life to simplify at-home self-monitoring and reduce human errors.

Existing literature in lung sound recognition and characterization includes algorithms, pipelines, and methodologies for cough detection, wheeze recognition, respiration phase characterization, onset detection of drug inhalation, and asthma detection using speech signals. Most studies propose a combination of normalization, filtering, and pre-processing to emphasize the sound of interest within the recorded respiratory sounds. Mel Frequency Cepstral Coefficients (MFCCs) and Linear Predictive Coding (LPC) coefficients are typically employed as features. Most recent works rely on supervised learning techniques to train linear classifiers, Gaussian models, or shallow neural networks tailored to the downstream task.

Although early studies demonstrate the existence of asthma fingerprints in audio signals, the experiments are typically performed on small datasets, with limited scope, and with specialized equipment (e.g. stethoscopes or wearable devices), which becomes a challenge to scale to an in-home concept that could seamlessly integrate into a patient's life. Moreover, the inventors of this disclosure have observed radically different lung sounds when recorded via a non-contact microphone versus a stethoscope. In particular, for some patients such as pediatric patients, soft sounds such as breathing are almost indistinguishable within the background noise when collected with a non-contact microphone, while wheezing sounds are also not as well identifiable when compared to contact stethoscope acquisitions.

Despite the clear advantage of creating an automatic lung sound assessment system for asthma monitoring, the field remains under-explored due to a lack of large scale data availability. Collecting lung sound data from asthmatic patients was limited in the literature due to: (1) the required expertise and complexity of the necessary hardware, often in the form of multiple contact sensors or stethoscope auscultation, (2) the chronic nature of asthma which requires long-term data recordings, and (3) expensive data annotation cost of the rare lung sound abnormalities during long recordings, requiring the expertise of specialized physicians or trained individuals. Increasing the potential of audio-based data-driven approaches to support clinical decision making in healthcare applications hinges on collecting and annotating new datasets in a reasonable time frame from large and diverse populations is critical to train robust and generalizable models.

In short, existing audio-based asthma monitoring solutions rely on feature engineering designs paired with contact-based auscultation which are brittle in practice and do not scale beyond point of care setups. Data-driven methods utilizing contactless microphones have the potential to address such limitations. However, these solutions are under-explored in healthcare due to, among other things, the high cost of data curation requiring physicians-in-the-loop. Therefore the present disclosure proposes an active learning (AL) system to facilitate audio data collection and annotation. The system detects lung sound abnormalities in asthma. AL reduces the annotation cost while increasing the model performance under a constrained annotation budget. It automatically extracts interesting audio segments from the continuous recordings, and efficiently annotates and trains anomaly detector model. The experimental results confirm the effectiveness of the proposed system as an enabler for larger scale data curation on a newly collected audio corpus for pediatric asthma.

According to embodiments disclosed herein, the inventors provide a system to enable large audio data collection and annotation based on active learning (AL) for assessing lung sound abnormalities. In some embodiments, the disclosed system is made of three main components: (1) a pool of unlabeled data from continuous audio recordings - from a controlled or in-the-wild data collection setup; (2) automatic data segmentation to extract audio chunks containing relevant audio events or sounds of interest from the unlabeled data stream; and (3) an AL pipeline to efficiently annotate the data while reducing the human effort and increasing the modeling performance under a constrained annotation budget. While the system executes on a pool of audio data collected for a pediatric asthma use case, the proposed framework enables more scalable data collection and annotation in other audio abnormality detection tasks in the healthcare domain. This is because active-learning frameworks in audio health care applications is completely lacking in the field.

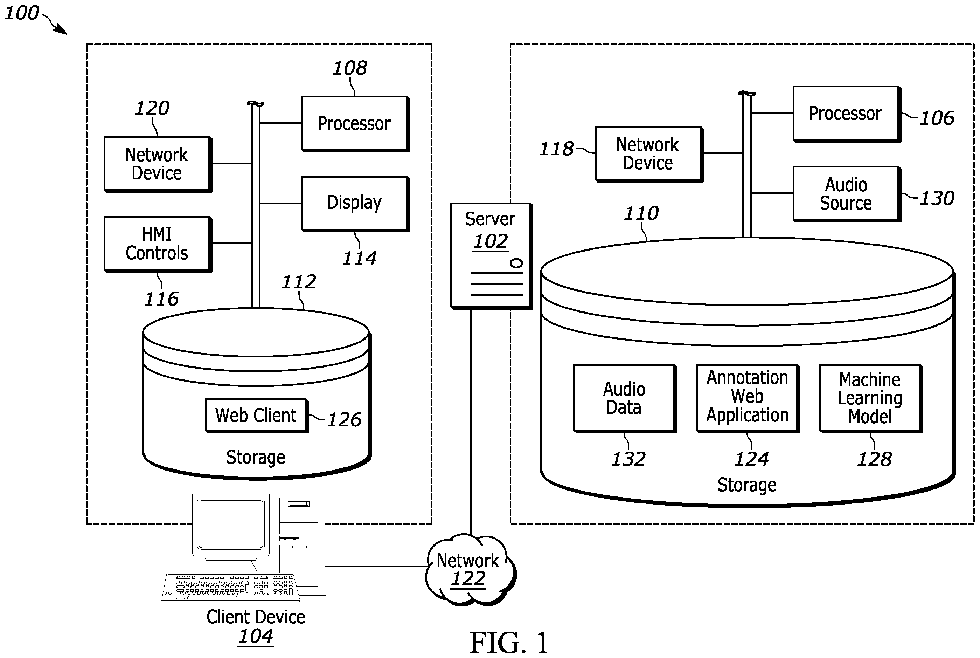

FIG. 1 illustrates an example active learning system 100 configured to carry out the large scale audio collection and annotation of audio data 132 via active learning described herein. The system 100, also referred to as an annotation system, includes a server 102 that hosts an annotation web application 124 that is accessible to client devices 104 over a network 122. The server 102 includes a processor 106 that is operatively connected to a storage 110 and to a network device 118. The server 102 further includes an audio data input source 130 for the receipt of audio data 132. The client device 104 includes a processor 108 that is operatively connected to a storage 112, a display device 114, human-machine interface (HMI) controls 116, and a network device 120. It should be noted that the example annotation system 100 is one example, and other systems 100 may be used. For instance, while only one client device 104 is shown, systems 100 including multiple client devices 104 are contemplated. As another possibility, while the example implementation is shown as a web-based application, alternate systems may be implemented as standalone systems or as client-server systems with thick client software. The client device 104 can be communicatively coupled to the server 102 in other methods.

Each of the processor 106 of the server 102 and the processor 108 of the client device 104 may include one or more integrated circuits that implement the functionality of a central processing unit (CPU) and/or graphics processing unit (GPU). In some examples, the processors 106, 108 are a system on a chip (SoC) that integrates the functionality of the CPU and GPU. The SoC may optionally include other components such as, for example, the storage 110 and the network device 118 or 120 into a single integrated device. In other examples, the CPU and GPU are connected to each other via a peripheral connection device such as PCI express or another suitable peripheral data connection. In one example, the CPU is a commercially available central processing device that implements an instruction set such as one of the x86, ARM, Power, or MIPS instruction set families.

Regardless of the specifics, during operation, the processors 106, 108 execute stored program instructions that are retrieved from the storages 110, 112, respectively. The stored program instructions accordingly include software that controls the operation of the processors 106, 108 to perform the operations described herein. The storages 110, 112 may include both non-volatile memory and volatile memory devices. The non-volatile memory includes solid-state memories, such as NAND flash memory, magnetic and optical storage media, or any other suitable data storage device that retains data when the annotation system 100 is deactivated or loses electrical power. The volatile memory includes static and dynamic random-access memory (RAM) that stores program instructions and data during operation of the annotation system 100.

The GPU of the client device 104 may include hardware and software for display of at least two-dimensional (2D) and optionally three-dimensional (3D) graphics to a display device 114 of the client. The display device 114 may include an electronic display screen, projector, printer, or any other suitable device that reproduces a graphical display. The display device 114 may display the audio data in audio form, and therefore the dispay device 114 may be a speaker or headphones. In other embodiments, the display device can display the sound data, such as a spectrogram. In some examples, the processor 108 of the client 104 executes software programs using the hardware functionality in the GPU to accelerate the performance of machine learning or other computing operations described herein.

The HMI controls 116 of the client 104 may include any of various devices that enable the client device 104 of the annotation system 100 to receive control input from workers or other users. Examples of suitable input devices that receive human interface inputs may include keyboards, mice, microphones, trackballs, touchscreens, voice input devices, graphics tablets, and the like.

The network devices 118, 120 may each include any of various devices that enable the server 102 and client device 104, respectively, to send and/or receive data from each other and external devices over the network 122. Examples of suitable network devices 118, 120 include a network adapter or peripheral interconnection device that receives data from another computer or external data storage device, which can be useful for receiving large sets of data in an efficient manner.

The annotation web application 124 be an example of a software application executed by the server 102. When executed, the annotation web application 124 may use various algorithms to perform aspects of the operations described herein. In an example, the annotation web application 124 may include instructions executable by the processor 106 of the server 102 as discussed above. Computer-executable instructions may be compiled or interpreted from computer programs created using a variety of programming languages and/or technologies, including, without limitation, and either alone or in combination, JAVA, C, C++, C#, VISUAL BASIC, JAVASCRIPT, PYTHON, PERL, PL/SQL, etc. In general, the processor 106 receives the instructions, e.g., from the storage 110, a computer-readable medium, etc., and executes these instructions, thereby performing one or more processes, including one or more of the processes described herein. Such instructions and other data may be stored and transmitted using a variety of computer-readable media.

The web client 126 may be a web browser, or other web-based client, executed by the client device 104. When executed, the web client 126 may allow the client device 104 to access the annotation web application 124 to display user interfaces of the annotation web application 124. The web client 126 may further provide input received via the HMI controls 116 to the annotation web application 124 of the server 102 over the network 122. Again, in other embodiments the web client 126 is a standalone systems or client-server systems with thick client software.

In artificial intelligence (AI) or machine learning systems, model-based reasoning refers to an inference method that operates based on a machine learning model 128 of a worldview to be analyzed. Generally, the machine learning model 128 is trained to learn a function that provides a precise correlation between input values and output values. At runtime, a machine learning engine uses the knowledge encoded in the machine learning model 128 against observed data to derive conclusions such as a diagnosis or a prediction. One example machine learning system may include the Deviation Network (DevNet) introduced in Deep Anomaly Detection with Deviation Networks, authored by G. Pang, C. Shen and A. van den Hangel in Proceedings of the 25th ACM SIGKDD International Conference on Knowledge Discovery and Data Mining in 2019, cited to by Applicant. DevNet is a few-shot anomaly detection method for image applications. DevNet is a semi-supervised method that predicts anomaly scores in an end-to-end manner versus previous works that learn representative features to separate normal vs. abnormal examples. This makes DevNet a great sampling strategy and strong candidate to be used in the AL system disclosed herein, however other machine learning models can be used. The DevNet network is first trained unsupervised by assigning normal scores to all the training samples. Next, it defines an average anomaly score of the normal data based on prior probabilities (e.g., Gaussian distribution) as a reference score to guide the subsequent anomaly score learner. Then, DevNet introduces a bias loss to enforce statistically significant deviation of anomaly scores of the abnormal data, while having the scores of the normal data close to the mean of the Gaussian prior. As discussed in detail herein, the annotation web application 124 and machine learning model 128 may be configured to recognize and annotate features of the audio data 132 for use in an Active Learning system to rely on a human user to decide whether an audio event should be classified as asthma or not, and the audio events selected to handing to the human for annotation is selected based on a confidence score of that audio event output by the machine learning model 128.

The audio data source 130 may be a microphone for example. In another example, the audio data input 132 may be an interface, such as the network device 118 or an interface to the storage 110, for the retrieval of previously-captured audio data 132. In another embodiment, the audio data source 130 is an ultrasonic device, and the audio data is ultrasonic data that can be visualized on a screen for example.

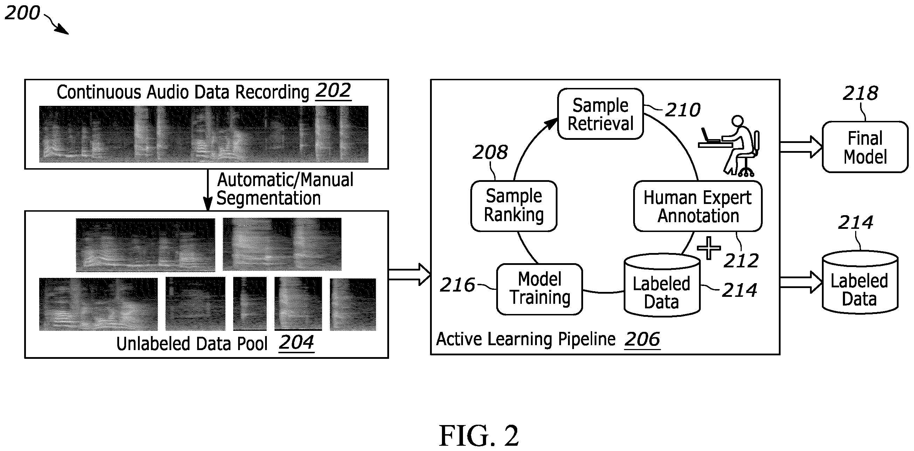

FIG. 2 illustrates the overall system 200 for enabling large scale audio data collection and annotation for healthcare applications via an Active Learning (AL) framework. The system 200 can rely on the system 100 described above for execution. The system 200 includes continuous audio data collection in-the-wild (e.g., at-home, doctor's office, etc.) using far-field non-contact microphone(s) to reduce the need for specialized sensors (e.g., stethoscope). Depending on the dataset size, the continuous data is manually segmented into audio chunks and/or pre-trained models can be utilized to automatically extract the desired audio segments. Then, the generated unlabeled pools or chunks of audio samples in the AL pipeline are used to efficiently annotate the target audio events, thus capitalizing on expert annotators' (e.g., physicians) time with a limited annotation budget. Details of these components are described further herein.

For example, in an embodiment audio data is captured, recorded, and stored at 202. Audio signals from an audio capturing device (e.g., microphone) associate with a medical device for detecting asthma (for example) can be captured when the medical device is in use. The audio samples can be a collected in collaboration with clinicians, for example after institutional review board (IRB) approval. The data can be collected in a physician's office after the patient's appointment, under typical noise conditions of a healthcare facility. The collected data can include audio associated with respiratory conditions such as breathing and activity associated with healthy (non-asthma) patients, non-asthma patients with a respiratory cold or illness, and asthma patients. Additional information such as height, weight, BMI, temperature, medications, and gender can also be provided to the system and associated with each audio sample. In other embodiments, the audio is not continuously captured and stored, but rather only captured and/or stored when an operator actuates the system.

In an embodiment, the audio data is recorded at a sampling rate of 44.1 kHz for a range of tasks with a portable hardware setup of a far-field microphone connected to a laptop and placed on a desk roughly zero to one meters away from the patients. The clinician can then ask the patient to perform various tasks while being recorded, such as (1) reciting the ABCs, (2) counting numbers one to ten, (3) saying "ahh", (4) naming two favorite colors, (5) naming favorite foods, (6) coughing, (7) taking deep breaths, (8) sitting silent. During deep breathing and sitting silent, a digital stethoscope can be used to collect ground truth data accompanying microphone data.

The audio recordings can then be segmented and labeled for each task by automatic or human annotators. The quality of the segments is then verified by an independent annotator. The obtained segments can have variable lengths depending on the nature of the task and the individual participants.

The audio recordings at 202 can be automatically or manually segmented into an unlabeled data pool 204. During automatic segmentation, the system 200 can use a pre-trained model to extract target audio events from a stream of data for later processing steps. For this study, pre-trained audio neural networks (PANNs) can be executed to propose segments for further processing. PANNs provide convolutional neural networks (CNNs) based architectures and are trained (supervised) on audio data such as AudioSet a massive general audio dataset containing 1.9 million audio clips with an ontology of 527 sound classes, including some relevant to clinical audio-based biomarkers (e.g. coughing, breathing, heart sounds, etc.). The system can then execute a pre-trained CNN model (e.g., Cnn14 DecisionLevelMax), outputting frame-wise sound event detection (SED) scores for all 527 classes. The predictions of our target events are taken and subjected to a threshold for binary predictions. The system then groups consecutive positive predictions to acquire the information of onsets and offsets for each segment of the desired sound events.

The pool of sound segments can then be fed into an active learning system 206. Active learning is a machine learning training technique that focuses on improving model performance by intelligently selecting the most informative data points to be labeled by an oracle (a human expert or a domain-specific system). Active learning actively chooses the most valuable samples for labeling, which helps the machine learning model 128 learn more efficiently and with fewer labeled examples compared to traditional supervised learning. In particular, AL is a machine learning solution that prioritizes the data to be labeled for optimizing the labeling cost while increasing the model robustness for use cases in which data annotation is costly and time consuming. In a typical supervised learning scenario, a model is trained on a fixed dataset with fully labeled examples. However, acquiring labeled data can be expensive and time-consuming, especially in cases where expert labeling is required. Active learning aims to address this limitation by actively involving the learning algorithm in the data labeling process.

As shown in FIG. 2, the AL framework includes the following iterative steps, according to an embodiment. First, at 208 the system applies an unsupervised/semi-supervised ranking method to sort unlabeled data samples (e.g., from 204) by assigning higher scores to more informative samples. For example, the an audio-based machine learning model 128 (e.g., DevNet) that is configured to determine what, if any, medical condition is associated with a sound can be executed on the unlabeled data in order to output a predicted label or associated medical condition, along with a confidence score representing the model's confidence in the predicted label or associated medical condition. Audio segments with high confidence scores (e.g., 90% or higher) are sorted from audio segments with lower confidence scores. The confidence scores represent the model's confidence that the audio sample is labeled as a certain event (e.g., asthma patient breathing).

At 210, the system retrieves a subset of the ranked samples. This can be, for example, based on the available annotation budget *B* of the expert human annotator. The samples can be retrieved based on their confidence score. For example, the system might only retrieve samples with confidence scores under a certain threshold (e.g., 80%). This threshold can be modified for various system designs and constraints. In other embodiments, the system retrieves a subset of samples based on diversity sampling such that the selected sounds given to the human for annotation are diverse in class or characteristics.

At 212, the human expert annotator performs an annotation on the selected audio samples. The human annotator might label a sound as cough, asthma, sneeze, or the like associated with respiration. The human annotator might label a sound as a heart murmur or other abnormal heart sound, or normal heart sound. The human annotator might also label a sound as an abnormal or normal bowel sound. The human's annotation can be stored so that the data can now be labeled, with the label being provided not from the machine learning model but from the human. The machine learning model 128 (e.g., DevNet) can then be trained or fine-tuned at 216 based on the collected annotations and the available unlabeled data at 216.

The steps in the active learning pipeline 206 can be repeated or reiterated until the machine learning model 128 achieves a desired model performance, e.g. convergence. It should be understood that in this disclosure, "convergence" can mean a set (e.g., predetermined) number of iterations have occurred, or that the residual is sufficiently small (e.g., the change in the approximate probability over iterations is changing by less than a threshold), or other convergence conditions. Once convergence is achieved, this yields a final, trained machine learning model at 218. In healthcare applications, the system will look for the presence of anomalies in the audio data, such as the presence of wheezing during cough, or a murmur in a heart sound. To aid in this type of detection, the system integrates DevNet or other few-shot anomaly detection models 128 in the AL pipeline as described above.

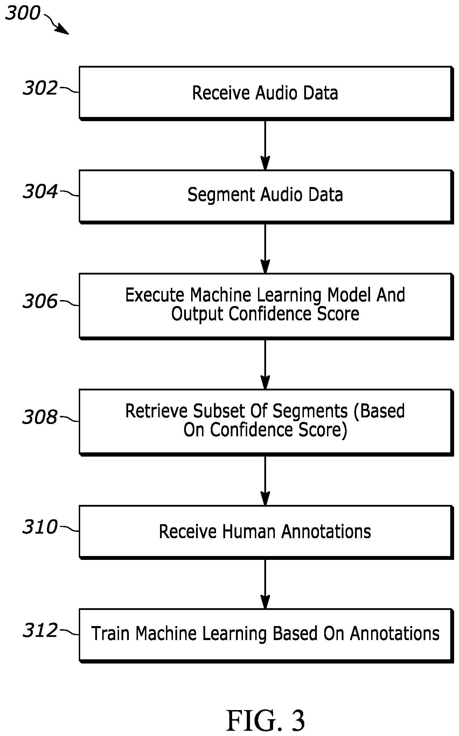

FIG. 3 illustrates a method 300 of training a machine learning model with the active learning pipeline described above, according to an embodiment. The method may be carried out using the structure of FIG. 1 and the in view of the framework shown in FIG. 2. At 302, the system receives audio data. This can be a continuous audio data recording 202, or some other audio data that is not labeled. For example, the audio data can be an unlabeled data pool 204 that is already automatically or manually segmented. The data can be generated from a microphone either at a clinician's office or at the patient's home. In the example in which the microphone is at the patient's home, the microphone can be part of a voice-integrated smart speaker device (e.g., AMAZON ECHO, GOOGLE HOME, etc.). When the machine learning model is eventually executed on this data, it can alert the patient in their own home when a sound might indicate the presence of a medical condition worthy of attention. For example, the microphone can generate sound data throughout the day, including all sorts of noise such as talking, walking, breathing, and normal household noises. If, however, some of those noises are determined to be indicative of asthma or another medical condition, the trained machine learning model can provide the user with an audio or visual alert indicating the presence of an asthmatic event. In any event, this sort of end-use of the system is first trained via active learning which is described herein.

At 304, if the audio data is not already segmented, the system can segment the audio data. The segmenting of the audio data can be either manual (e.g., human) or automatic (e.g., via a ML model). During this step, the system can remove chunks of audio that are silent or irrelevant, and thus not important for inclusion into the audio-based machine learning model 128. The system can also cut or splice the audio stream into segments, with each segment corresponding to an audio event, such as one that might indicate a caught, sneeze, breath, yell, spoken word, heartbeat(s), or the like. If done automatically, this can be performed with a PANN or the like as described above.

At 306, the system executes a machine learning model 128 on the audio segments. The machine learning model 128 can be audio-based and can include a network such as DevNet or others as explained herein. The machine learning model 128 is configured to output, for each audio segment, a label of a medical condition and an associated a confidence score. For example, the machine learning model 128 can be executed with audio data associated with one audio segment, and conclude that the particular breathing in that segment is associated with asthma, with a 97% confidence. In another example, the machine learning model 128 can be executed with audio data associated with another audio segment, and conclude that the particular heartbeat pattern in that segment is associated with a heart murmur, with a 72% confidence. These outputted labels and confidence scores can be stored, e.g. in storage 110 for later retrieval.

At 308, the system retrieves from storage a subset of these segments for human-in-the-loop augmentation via active learning, as described with reference to FIG. 2. In one embodiment, the audio segments chosen at 308 are chosen based on their confidence scores and/or their label. For example, audio scores associated with an asthmatic event with a confidence score under a certain threshold (e.g. 85%) can be retrieved from storage and prepared for human annotation or confirmation of the asthmatic event. The confidence score threshold can be set depending on the amount of data desired to be fed to humans, and can vary based on the health condition.

The retrieved sound segments can then be provided to a human for annotation. For example, the human (e.g., doctor, clinician) can listen to the sound segment and indicate whether that sound is associated with a certain health condition such as asthma or others discussed herein. The human can label the sound data, or can confirm (or protest) the output of the machine-learning model if that model has already labeled the audio segment. At 310, the system receives these human annotations. The system can then update the machine learning model and the stored labels associated with that particular audio segment.

At 312, the machine learning model 128 is trained (or re-trained) with these human annotations. This can improve the accuracy of the outputs of the model, and ultimately its confidence scores, resulting in less audio samples needed to be annotated by humans. This process can continue until convergence, for example, which yields a trained audio-based machine learning model configured to determine the presence of a particular health condition associated with a certain audio event.

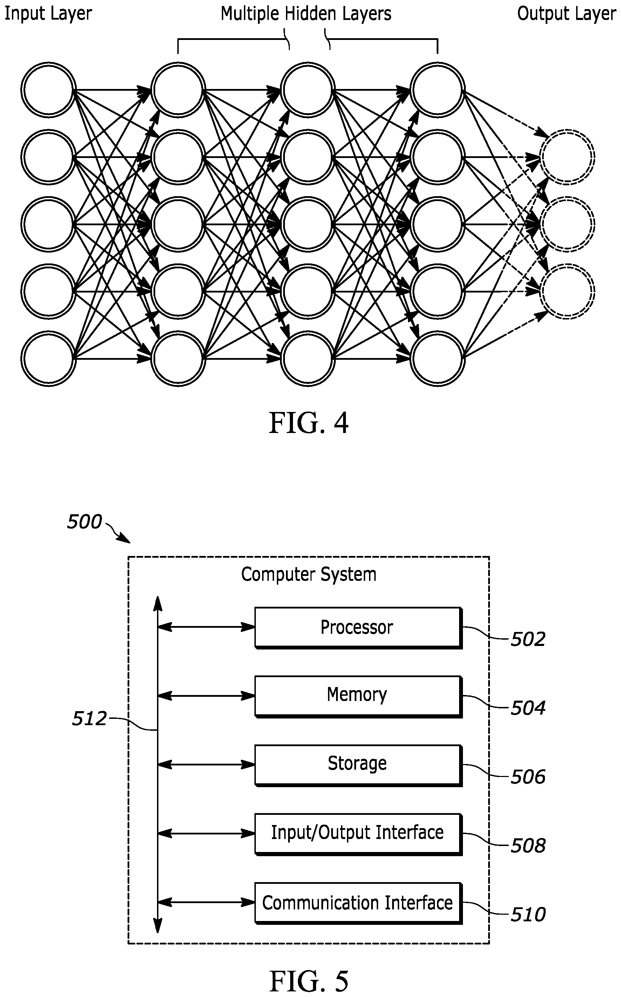

The various machine learning networks described herein, such as machine learning model 128, DevNet, final model 218, etc. can be or include a neural network, such as illustrated in the embodiment of FIG. 4. The models described herein can be a neural network (e.g., and in some cases, while not required, a deep neural network). The models may be configured as a data-oriented audio processing model that uses a data-oriented approach to classify or label a sound. The models can include an input layer (having a plurality of input nodes) and an output layer (having a plurality of output nodes). In some examples, the models may include a plurality of hidden layers. The nodes of the input layer, output layer, and hidden layers may be coupled to nodes of subsequent or previous layers. And each of the nodes of the output layer may execute an activation function - e.g., a function that contributes to whether the respective nodes should be activated to provide an output of the model. The quantities of nodes shown in the input, hidden, and output layers are merely exemplary and any suitable quantities may be used.

FIG. 5 illustrates an example computer system 500. In particular embodiments, one or more computer systems 500 perform one or more steps of one or more methods described or illustrated herein, and can be used as, or in conjunction with, the systems illustrated in FIG. 1-2. In particular embodiments, one or more computer systems 500 provide functionality described or illustrated herein. In particular embodiments, software running on one or more computer systems 500 performs one or more steps of one or more methods described or illustrated herein or provides functionality described or illustrated herein. Particular embodiments include one or more portions of one or more computer systems 500. Herein, reference to a computer system may encompass a computing device, and vice versa, where appropriate. Moreover, reference to a computer system may encompass one or more computer systems, where appropriate.

This disclosure contemplates any suitable number of computer systems 500. This disclosure contemplates computer system 500 taking any suitable physical form. As example and not by way of limitation, computer system 500 may be an embedded computer system, a system-on-chip (SOC), a single-board computer system (SBC) (such as, for example, a computer-on-module (COM) or system-on-module (SOM)), a desktop computer system, a laptop or notebook computer system, an interactive kiosk, a mainframe, a mesh of computer systems, a mobile telephone, a personal digital assistant (PDA), a server, a tablet computer system, or a combination of two or more of these. Where appropriate, computer system 500 may include one or more computer systems 500; be unitary or distributed; span multiple locations; span multiple machines; span multiple data centers; or reside in a cloud, which may include one or more cloud components in one or more networks. Where appropriate, one or more computer systems 500 may perform without substantial spatial or temporal limitation one or more steps of one or more methods described or illustrated herein. As an example and not by way of limitation, one or more computer systems 500 may perform in real time or in batch mode one or more steps of one or more methods described or illustrated herein. One or more computer systems 500 may perform at different times or at different locations one or more steps of one or more methods described or illustrated herein, where appropriate.

In particular embodiments, computer system 500 includes a processor 502, memory 504, storage 506, an input/output (I/O) interface 508, a communication interface 510, and a bus 512. Although this disclosure describes and illustrates a particular computer system having a particular number of particular components in a particular arrangement, this disclosure contemplates any suitable computer system having any suitable number of any suitable components in any suitable arrangement.

In particular embodiments, processor 502 includes hardware for executing instructions, such as those making up a computer program. As an example and not by way of limitation, to execute instructions, processor 502 may retrieve (or fetch) the instructions from an internal register, an internal cache, memory 504, or storage 506; decode and execute them; and then write one or more results to an internal register, an internal cache, memory 504, or storage 506. In particular embodiments, processor 502 may include one or more internal caches for data, instructions, or addresses. This disclosure contemplates processor 502 including any suitable number of any suitable internal caches, where appropriate. As an example and not by way of limitation, processor 502 may include one or more instruction caches, one or more data caches, and one or more translation lookaside buffers (TLBs). Instructions in the instruction caches may be copies of instructions in memory 504 or storage 506, and the instruction caches may speed up retrieval of those instructions by processor 502. Data in the data caches may be copies of data in memory 504 or storage 506 for instructions executing at processor 502 to operate on; the results of previous instructions executed at processor 502 for access by subsequent instructions executing at processor 502 or for writing to memory 504 or storage 506; or other suitable data. The data caches may speed up read or write operations by processor 502. The TLBs may speed up virtual-address translation for processor 502. In particular embodiments, processor 502 may include one or more internal registers for data, instructions, or addresses. This disclosure contemplates processor 502 including any suitable number of any suitable internal registers, where appropriate. Where appropriate, processor 502 may include one or more arithmetic logic units (ALUs); be a multi-core processor; or include one or more processors 502. Although this disclosure describes and illustrates a particular processor, this disclosure contemplates any suitable processor.

In particular embodiments, memory 504 includes main memory for storing instructions for processor 502 to execute or data for processor 502 to operate on. As an example and not by way of limitation, computer system 500 may load instructions from storage 506 or another source (such as, for example, another computer system 500) to memory 504. Processor 502 may then load the instructions from memory 504 to an internal register or internal cache. To execute the instructions, processor 502 may retrieve the instructions from the internal register or internal cache and decode them. During or after execution of the instructions, processor 502 may write one or more results (which may be intermediate or final results) to the internal register or internal cache. Processor 502 may then write one or more of those results to memory 504. In particular embodiments, processor 502 executes only instructions in one or more internal registers or internal caches or in memory 504 (as opposed to storage 506 or elsewhere) and operates only on data in one or more internal registers or internal caches or in memory 504 (as opposed to storage 506 or elsewhere). One or more memory buses (which may each include an address bus and a data bus) may couple processor 502 to memory 504. Bus 512 may include one or more memory buses, as described below. In particular embodiments, one or more memory management units (MMUs) reside between processor 502 and memory 504 and facilitate accesses to memory 504 requested by processor 502. In particular embodiments, memory 504 includes random access memory (RAM). This RAM may be volatile memory, where appropriate. Where appropriate, this RAM may be dynamic RAM (DRAM) or static RAM (SRAM). Moreover, where appropriate, this RAM may be single-ported or multi-ported RAM. This disclosure contemplates any suitable RAM. Memory 504 may include one or more memories 504, where appropriate. Although this disclosure describes and illustrates particular memory, this disclosure contemplates any suitable memory.

In particular embodiments, storage 506 includes mass storage for data or instructions. As an example and not by way of limitation, storage 506 may include a hard disk drive (HDD), a floppy disk drive, flash memory, an optical disc, a magneto-optical disc, magnetic tape, or a Universal Serial Bus (USB) drive or a combination of two or more of these. Storage 506 may include removable or non-removable (or fixed) media, where appropriate. Storage 506 may be internal or external to computer system 500, where appropriate. In particular embodiments, storage 506 is non-volatile, solid-state memory. In particular embodiments, storage 506 includes read-only memory (ROM). Where appropriate, this ROM may be mask-programmed ROM, programmable ROM (PROM), erasable PROM (EPROM), electrically erasable PROM (EEPROM), electrically alterable ROM (EAROM), or flash memory or a combination of two or more of these. This disclosure contemplates mass storage 506 taking any suitable physical form. Storage 506 may include one or more storage control units facilitating communication between processor 502 and storage 506, where appropriate. Where appropriate, storage 506 may include one or more storages 506. Although this disclosure describes and illustrates particular storage, this disclosure contemplates any suitable storage.

In particular embodiments, I/O interface 508 includes hardware, software, or both, providing one or more interfaces for communication between computer system 500 and one or more I/O devices. Computer system 500 may include one or more of these I/O devices, where appropriate. One or more of these I/O devices may enable communication between a person and computer system 500. As an example and not by way of limitation, an I/O device may include a microphone, keyboard, keypad, microphone, monitor, mouse, printer, scanner, speaker, still camera, stylus, tablet, touch screen, trackball, video camera, another suitable I/O device or a combination of two or more of these. An I/O device may include one or more sensors. This disclosure contemplates any suitable I/O devices and any suitable I/O interfaces 508 for them. Where appropriate, I/O interface 508 may include one or more device or software drivers enabling processor 502 to drive one or more of these I/O devices. I/O interface 508 may include one or more I/O interfaces 508, where appropriate. Although this disclosure describes and illustrates a particular I/O interface, this disclosure contemplates any suitable I/O interface.

In particular embodiments, communication interface 510 includes hardware, software, or both providing one or more interfaces for communication (such as, for example, packet-based communication) between computer system 500 and one or more other computer systems 500 or one or more networks. As an example and not by way of limitation, communication interface 510 may include a network interface controller (NIC) or network adapter for communicating with an Ethernet or other wire-based network or a wireless NIC (WNIC) or wireless adapter for communicating with a wireless network, such as a WI-FI network. This disclosure contemplates any suitable network and any suitable communication interface 510 for it. As an example and not by way of limitation, computer system 500 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As an example, computer system 500 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination of two or more of these. Computer system 500 may include any suitable communication interface 510 for any of these networks, where appropriate. Communication interface 510 may include one or more communication interfaces 510, where appropriate. Although this disclosure describes and illustrates a particular communication interface, this disclosure contemplates any suitable communication interface.

In particular embodiments, bus 512 includes hardware, software, or both coupling components of computer system 500 to each other. As an example and not by way of limitation, bus 512 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination of two or more of these. Bus 512 may include one or more buses 512, where appropriate. Although this disclosure describes and illustrates a particular bus, this disclosure contemplates any suitable bus or interconnect.

Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

Although much of the disclosure provided above is focused on or discusses the use of active learning to detect asthma, this disclosure should not be limited to only asthma. Instead, the teachings provided herein can be applied to other respiratory conditions, such as chronic obstructive pulmonary disease (COPD), COVID (e.g., COVID-19), and the like. These respiratory conditions can be diagnosed by a doctor by listening to breathing, coughing, and wheezing patterns, and thus the applied learning system herein can utilize a clinician human-in-the-loop in the active learning system to properly label sound segments that indicate the patient as having one of these respiratory conditions.

The teachings provided herein can be applied to other human biological sounds, such as sounds emitted by the human heart or bowels. For example, by listening to the sounds emitted by the heart, the machine learning models disclosed herein can be trained via active learning and human-in-the-loop to properly diagnose a heart condition, such as a heart murmur or the like. Heart sounds can be described by their intensity, pitch, quality, and timing. Sounds associated with the heart captured by a microphone (e.g., attached to or in communication with a stethoscope or the like) can be labeled by a human clinician in the active learning systems described herein.

As another example, the teachings herein can be applied to sounds emitted by human bowels. It is common for anesthesia to place a patient's bowels in a "sleep" mode. Therefore, in some post-op scenarios after surgery, a doctor will listen to determine if the patient's digestive system is functioning properly. These sounds can be used in the active learning system such that a clinician human-in-the-loop can label these sounds to better train the machine learning model to automatically determine if the sounds produced by the patient's bowels are normal or abnormal.

These are just some examples of biological sounds produced by the human body that can benefit from the teachings herein. The teachings provided herein can be applied to any biological sound produced by the human that would enable a human clinician (and therefore a machine learning model) to determine a medical condition associated with such sound.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

## Claims

1. A method for training an audio-based machine learning model with active learning, the method comprising:
receiving audio data corresponding to biological sounds produced by a body of a patient;
segmenting the audio data into a plurality of segments;
executing an audio-based machine learning model on the plurality of segments, wherein the audio-based machine learning model is configured to output, for each segment, a label of a medical condition and an associated a confidence score;
storing the labels and associated confidence scores in storage; and
training the audio-based machine learning model via active learning, wherein the training includes:
retrieving a subset of the plurality of segments from the storage;
receiving annotations from a human annotator, wherein the annotations are associated with medical conditions; and
training the audio-based machine learning model based on the annotations until convergence to yield a trained sound-based machine learning model configured to output a predicted medical condition associated with input biological sounds.

2. The method of claim 1, wherein the segmenting is performed via a pre-trained audio neural network (PANN), and each of the plurality of segments is associated with a respective audio event corresponding to an audio-based biomarker.

3. The method of claim 1, wherein the plurality of segments are each associated with breathing, the predicted medical condition includes asthma.

4. The method of claim 1, wherein the plurality of segments are each associated with heartbeats, the predicted medical condition includes a heart murmur.

5. The method of claim 1, wherein the subset of the plurality of segments are selected for retrieval based upon confidence scores associated with the plurality of segments being under a threshold.

6. The method of claim 1, wherein the microphone is attached to a stethoscope.

7. The method of claim 1, wherein the audio data is a continuous stream of audio data.

8. A system comprising:
a processors; and
a non-transitory memory coupled to the processor comprising instructions executable by the processor, the processor operable when executing the instructions to:
receive audio data generated from a microphone and corresponding to biological sounds produced by a body of a patient;
segment the audio data into a plurality of segments;
execute an audio-based machine learning model on the plurality of segments, wherein the audio-based machine learning model is configured to output, for each segment, a label of a medical condition and an associated a confidence score;
store the labels and associated confidence scores; and
train the audio-based machine learning model via active learning, wherein the training includes:
retrieving a subset of the plurality of segments;
receiving annotations from a human annotator, wherein the annotations are associated with medical conditions; and
training the audio-based machine learning model based on the annotations until convergence to yield a trained sound-based machine learning model configured to output a predicted medical condition.

9. The system of claim 8, wherein the audio data is a continuous stream of audio data.

10. The system of claim 9, wherein the segmenting of the audio data is performed via a pre-trained audio neural network (PANN), and each of the plurality of segments is associated with a respective audio event corresponding to an audio-based biomarker.

11. The system of claim 8, wherein the plurality of segments are each associated with breathing, the predicted medical condition includes asthma.

12. The system of claim 8, wherein the plurality of segments are each associated with heartbeats, the predicted medical condition includes a heart murmur.

13. The system of claim 8, wherein the subset of the plurality of segments are selected for retrieval based upon confidence scores associated with the plurality of segments being under a threshold.

14. The system of claim 8, wherein the microphone is attached to a stethoscope.

15. A computer-readable non-transitory storage medium embodying software that is operable, when executed, to:
receive audio data generated from a microphone and corresponding to biological sounds produced by a body of a patient;
segment the audio data into a plurality of segments,
execute an audio-based machine learning model on the plurality of segments, wherein the audio-based machine learning model is configured to output, for each segment, a label of a medical condition and an associated a confidence score;
store the labels and associated confidence scores; and
train the audio-based machine learning model via active learning, wherein the training includes:
retrieving a subset of the plurality of segments;
receiving annotations from a human annotator, wherein the annotations are associated with medical conditions; and
training the audio-based machine learning model based on the annotations until convergence to yield a trained sound-based machine learning model configured to output a predicted medical condition.

16. The computer-readable non-transitory storage medium of claim 15, wherein the audio data is a continuous stream of audio data.

17. The computer-readable non-transitory storage medium of claim 16, wherein the segmenting of the audio data is performed via a pre-trained audio neural network (PANN), and each of the plurality of segments is associated with a respective audio event corresponding to an audio-based biomarker.

18. The computer-readable non-transitory storage medium of claim 15, wherein the plurality of segments are each associated with breathing, the predicted medical condition includes asthma.

19. The computer-readable non-transitory storage medium of claim 15, wherein the plurality of segments are each associated with heartbeats, the predicted medical condition includes a heart murmur.

20. The computer-readable non-transitory storage medium of claim 15, wherein the subset of the plurality of segments are selected for retrieval based upon confidence scores associated with the plurality of segments being under a threshold.
